(19) Europäisches Patentamt
European Patent Office
Office européen des brevets

(11) Veröffentlichungsnummer: **0 532 565 B1**

# (12) EUROPÄISCHE PATENTSCHRIFT

(45) Veröffentlichungstag der Patentschrift: **23.11.94**

(51) Int. Cl.5: **C07J 21/00**, C07J 41/00, C07J 31/00

(21) Anmeldenummer: **91910278.0**

(22) Anmeldetag: **03.06.91**

(86) Internationale Anmeldenummer:
**PCT/EP91/01017**

(87) Internationale Veröffentlichungsnummer:
**WO 91/18917 (12.12.91 91/28)**

## (54) AUSGANGSVERBINDUNGEN FÜR DIE HERSTELLUNG VON 10-g(b)-H-STEROIDEN UND EIN VERFAHREN ZUR HERSTELLUNG DIESER AUSGANGSVERBINDUNGEN.

(30) Priorität: **01.06.90 DE 4018168**

(43) Veröffentlichungstag der Anmeldung:
**24.03.93 Patentblatt 93/12**

(45) Bekanntmachung des Hinweises auf die Patenterteilung:
**23.11.94 Patentblatt 94/47**

(84) Benannte Vertragsstaaten:
**AT BE CH DE DK ES FR GB GR IT LI LU NL SE**

(56) Entgegenhaltungen:
**WO-A-87/05908**
**WO-A-89/03390**
**US-A- 4 102 907**

(73) Patentinhaber: **SCHERING AKTIENGESELLSCHAFT**

**D-13342 Berlin (DE)**

(72) Erfinder: **OTTOW, Eckhard**
**Moltkestr. 48**
**D-1000 Berlin 45 (DE)**
Erfinder: **NEEF, Günter**
**Markgraf-Albrecht-Str. 4**
**D-1000 Berlin 31 (DE)**
Erfinder: **CLEVE, Arwed**
**Blumenthalstr. 19**
**D-1000 Berlin 42 (DE)**
Erfinder: **WIECHERT, Rudolf**
**Petzower Str. 8A**
**D-1000 Berlin 39 (DE)**

EP 0 532 565 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Verbindungen der allgemeinen Formel III

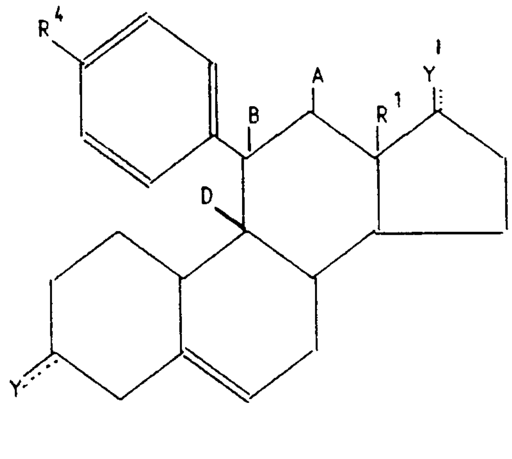

(III),

worin
A und B gemeinsam eine zusätzliche Bindung und
D ein Wasserstoffatom oder
B und D gemeinsam eine zusätzliche Bindung und
A ein Wasserstoffatom
bedeuten und
$R^4$ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten $C_1$-$C_8$-Alkyl-, -Acyl-oder Alkoxyalkylrest, für
eine Aminogruppe

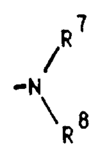

,

in welcher $R^7$ und $R^8$ unabhängig voneinander ein Wasserstoffatom oder eine $C_1$-$C_4$-Alkylgruppe bedeuten, oder für ein entsprechendes Aminoxid

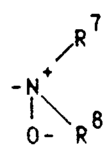

,

oder für die Gruppierungen $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα

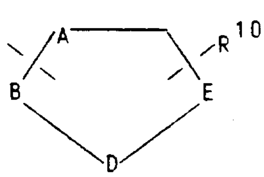

(Iα),

in welchem A ein Stickstoff-, Sauerstoff- oder Schwefelatom, -B-D-E- die Elementenfolge -C-C-C- , -N-C-C- oder -C-N-C- und $R^{10}$ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten $C_1$-$C_8$-Alkyl-, -Acyl-oder Alkoxyalkylrest,
eine Aminogruppe

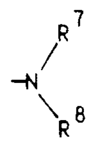 ,

in welcher $R^7$ und $R^8$ unabhängig voneinander ein Wasserstoffatom oder eine $C_1$-$C_4$-Alkylgruppe bedeuten, oder ein entsprechendes Aminoxid

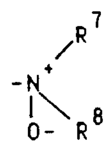 ,

oder die Gruppierung $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, symbolisieren,
oder für einen Heteroarylrest der Formel Iβ

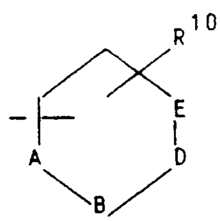

(Iβ),

in welchem A ein Stickstoffatom und -B-D-E- die Elementenfolge -C-C-C-, -N-C-C- , -C-N-C- oder -C-C-N- bedeuten und $R^{10}$ die bereits angegebene Bedeutung hat,
oder für einen Phenylrest der Formel Iγ

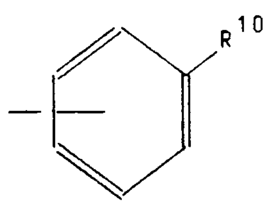

(Iγ),

worin $R^{10}$ die bereits angegebene Bedeutung hat,
stehen,
Y und Y' eine geschützte Ketogruppe oder eine geschützte Hydroxygruppe und ein Wasserstoffatom, wobei Y und Y' identisch oder verschieden sein können, sowie
$R^1$ eine Methyl- oder Ethylgruppe bedeuten,
ein Verfahren zu ihrer Herstellung und die für dieses Herstellungsverfahren benötigten Ausgangsprodukte.
Bevorzugt sind die folgenden Verbindungen:
3,3;17,17-Bis-(ethylendioxy)-11-phenyl-5,11-estradien,
3,3;17,17-Bis-(ethylendioxy)-11-(4-bromphenyl)-5,11-estradien.
3,3:17,17-Bis-(ethylendioxy)-11-(4-methoxyphenyl)-5,9(11)-estradien,
3,3;17,17-Bis-(ethylendioxy)-11-(4-methylphenyl)-5,9(11)-estradien,

3,3;17,17-Bis-(ethylendioxy)-11-phenyl-5,9(11)-estradien,
3,3;17,17-Bis-(ethylendioxy)-11-(4-bromphenyl)-5,9(11)-estradien,
3,3;17,17-Bis-(ethylendioxy)-11-(4-methoxyphenyl)-5,11-estradien,
3,3;17,17-Bis-(ethylendioxy)-11-(4-methylphenyl)-5,11-estradien,

Außer der Ethylendioxygruppe kann für Y/Y' beispielsweise auch die 2,2-Dimethylpropylen-1,3-dioxygruppe stehen. Auch andere gängige Schutzgruppen sind möglich. Steht Y und/oder Y' für eine geschützte Hydroxygruppe und ein Wasserstoffatom, kann die Hydroxygruppe beispielsweise als Methoxymethyl-, Methoxyethyl-, Tetrahydropyranyl- oder Silylether geschützt sein.

Die Verbindungen der allgemeinen Formel III selbst dienen als Ausgangsprodukte für das in der gleichzeitig eingereichten deutschen Patentanmeldung P 40 18 167.7 beschriebene neue Verfahren zur Herstellung der Endverbindungen der allgemeinen Formel I

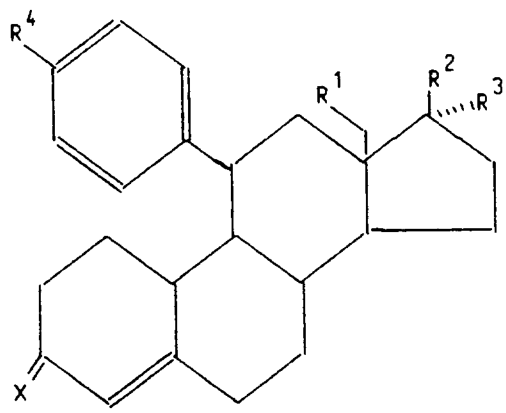

(I),

worin

- X für ein Sauerstoffatom, die Hydroxyiminogruppierung >N~OH oder zwei Wasserstoffatome,
- $R^1$ für ein Wasserstoffatom oder eine Methylgruppe,
- $R^2$ für eine Hydroxygruppe, eine $C_1$-$C_{10}$-Alkoxy- oder $C_1$-$C_{10}$-Acyloxygruppe,
- $R^3$ für ein Wasserstoffatom, die Gruppierung $-(CH_2)_nCH_2Z$, wobei n 0, 1, 2, 3, 4 oder 5 ist, Z ein Wasserstoffatom, die Cyanogruppe oder den Rest $-OR^5$ mit $R^5 = H$, $C_1$-$C_{10}$-Alkyl oder $C_1$-$C_{10}$-Acyl bedeuten, die Gruppierung $-(CH_2)_m-C\equiv C-Y$, wobei m 0, 1 oder 2 und Y ein Wasserstoff-, Fluor-, Chlor-, Brom- oder Jod-Atom, einen $C_1$-$C_{10}$-Hydroxyalkyl-, $C_1$-$C_{10}$-Alkoxyalkyl-, $C_1$-$C_{10}$-Acyloxyalkylrest bedeuten, die Gruppierung $-CH=CH-(CH_2)_kCH_2R^6$, wobei k 0, 1 oder 2 und $R^6$ ein Wasserstoffatom, eine Hydroxygruppe, einen $C_1$-$C_4$-Alkoxy- oder $C_1$-$C_4$-Acyloxyrest bedeuten,

  oder aber $R^2$ und $R^3$ gemeinsam für einen Rest der Formel

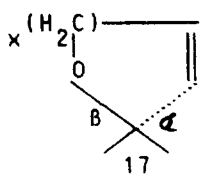

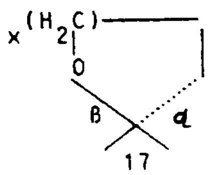

oder

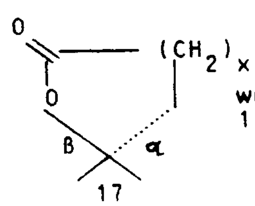

wobei x = 1 oder 2 ist

stehen,

sowie $R^4$ die bereits in der Formel III für diesen Substituenten angegebene Bedeutung hat.

Zu den Endverbindungen der allgemeinen Formel I gehören auch deren pharmakologisch verträgliche Additionssalze mit Säuren.

Die Verbindungen der allgemeinen Formel I selbst sind größtenteils bereits in der deutschen Patentanmeldung P 39 21 059.6 beschrieben. Sie besitzen in erster Linie wegen ihrer starken antigestagenen Eigenschaften großes Interesse.

Gemäß vorliegender Erfindung werden die Verbindungen der allgemeinen Formel III hergestellt, indem eine Verbindung der allgemeinen Formel IV

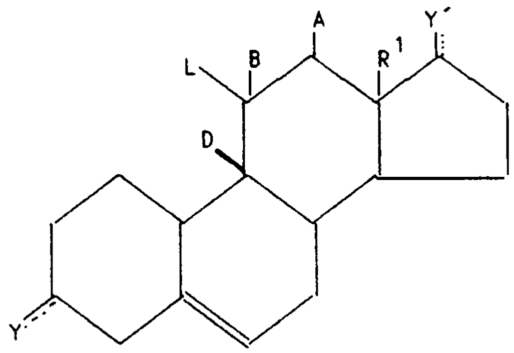

(IV),

worin A, B und D sowie Y und Y' und $R^1$ die in Formel III angegebene Bedeutung haben und L für eine Perfluoralkylsulfonyloxygruppe $C_nF_{2n+1}SO_2O$- (n = 1,2,3,4) steht, in Gegenwart einer katalytischen Menge eines Übergangsmetallkatalysators mit einer Arylverbindung der allgemeinen Formel V

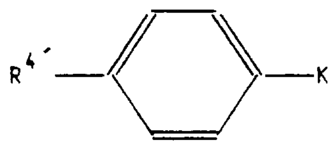

(V),

worin K für einen der Reste

-B(Alkyl)$_2$
-Sn(Alkyl)$_3$ Alkyl = $C_1$-$C_4$-Alkylrest
-B(OH)$_2$
-ZnHal
-MgHal Hal = Cl, Br, J

und $R^{4'}$ für einen der unter $R^4$ genannten Reste stehen,
zu einer Verbindung der allgemeinen Formel IIIa

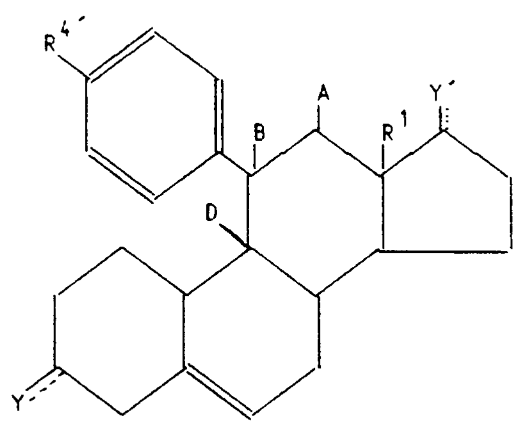

(IIIa),

worin A, B und D sowie Y und Y' und $R^1$ die in Formel III und $R^{4'}$ die in Formel V angegebene Bedeutung haben und gegebenenfalls, wenn $R^4$ in der Formel III eine andere Bedeutung als $R^{4'}$ in der Formel IIIa haben soll, eine Verbindung der allgemeinen Formel IIIa, worin $R^{4'}$ für ein Bromatom steht oder nach Überführung einer für $R^{4'}$ stehenden Methoxygruppe in eine Perfluoralkylsulfonyloxygruppe $C_nF_{2n+1}SO_2O$- (n = 1,2,3,4), mit einer Verbindung der allgemeinen Formel VI

$R^4$-K (VI),

worin $R^4$ die letztlich für diesen Substituenten in der Formel III gewünschte und K die bereits in Formel V angegebene Bedeutung haben,

umgesetzt wird.

Für L in der Verbindung der allgemeinen Formel IV steht vorzugsweise die Trifluormethylsulfonyloxygruppe.

Als Übergangsmetallkatalysator zur Kupplung der Arylverbindung der allgemeinen Formel V mit der die Abgangsgruppe L aufweisenden Verbindung dient gemäß den Beispielen vorliegender Erfindung Palladiumtetrakistriphenylphosphin (siehe nachstehend angegebene Literatur); genausogut könnte aber Nickeltetrakistriphenylphosphin oder ähnliche solche Übergangsmetallkatalysatoren verwendet werden.

Die Variante, daß der letztendlich gewünschte Substituent $R^4$ über die Funktionalisierung eines Brom- oder Methoxysubstituenten $R^{4'}$ in der Verbindung IIIa eingeführt wird, ist dann zu wählen, wenn die zu kuppelnde Arylverbindung der allgemeinen Formel V, worin $R^{4'}$ bereits mit $R^4$ identisch ist, nicht zugänglich oder zur Kupplung nicht geeignet ist.

Übergangsmetallkatalysierte Arylkupplungsreaktionen von Verbindungen des Typs der allgemeinen Formel V mit Verbindungen, die eine Abgangsgruppe tragen, sind beispielsweise beschrieben in: mit $-Sn(Alkyl)_3$-substituierten Aromaten: J.E. McMurry and S. Mohanraj, Tetrahydron Letters 24, No. 27, S. 2723-2726, 1983; X. Lu und J. Zhu, Communications, S. 726-727, 1987; Q.-Y. Chen und Z.-Y. Yang, Tetrahedron Letters 27, No 10, S. 1171-1174, 1986; S. Cacchi, P.G. Ciattini, E. Morera und G. Ortar, Tetrahedron Letters 27, No. 33, S. 3931-3934, 1986; A.M. Echavarren und J.K. Stille, J.Am.Chem.Soc. 1987, 109, S. 5478-5486 und J.Am.Chem.Soc. 1988, 110, S. 1557;

mit $-B(OH)_2$ und $-B(OAlkyl)_2$-substituierten Aromaten: Y. Hoshino, N. Miyaura und A. Suzuki, Bull. Chem. Soc. Jpn. 61, 3008 (1988); H. Matsubasa, K. Seto, T Tahara and S. Takahashi; Bull.Chem. Soc. Jpn, 62, 3896 (1989);

mit -ZnCl-substituierten Aromaten: R. McCague, Tet. Lett., 28, 701 (1987); A. Arcadi, A. Burini, S. Cacchi, M. Delmastro, F. Marinelli, B. Pietroni, Syn. Les., 1, 1990, S. 47.

Ist der Substituent $R^{4'}$ in der gekuppelten Verbindung der allgemeinen Formel IIIa nicht bereits der letztlich gewünschte Substituent $R^4$, so können Verbindungen der allgemeinen Formel IIIa in 4-Stellung des 11-Aromaten weiter funktionalisiert werden:

dazu wird entweder eine 4-Brom-phenyl-Verbindung IIIa mit einer Verbindung der allgemeinen Formel VI

$R^4$-K (VI)

umgesetzt (EP-A 0349 481, Seite 11) oder es wird eine 4-Methoxy-phenyl-Verbindung IIIa durch Spaltung des Methylethers beispielsweise mit Natriummethanthiolat und Veresterung der freien OH-Verbindung mit einem Perfluoralkylsulfonsäureanhydrid $(C_nF_{2n+1}SO_2)_2O$ $(n=1,2,3,4)$ (P.J. Stang, M. Hanack und L.R. Subramanian, Synthesis 85, (1982)) in die entsprechende 4-Perfluoralkylsulfonyloxyphenyl-Verbindung überführt und diese mit einer Verbindung der allgemeinen Formel VI reagieren gelassen. Die Kupplung der 4-Brom bzw. der 4-Perfluoralkylsulfonyloxy-phenyl-Verbindung mit der Verbindung der allgemeinen Formel VI erfolgt jeweils übergangsmetallkatalysiert nach den bereits vorstehend zitierten Methoden. Zahlreiche solcher Kupplungsreaktionen speziell an Steroiden, die eine Trifluormethansulfonyloxygruppe in der 4-Stellung des 11$\beta$-Phenylrings tragen, sind bereits in der EP-A 0 349 481 und EP-A 0 283 428 beschrieben.

Die für das hier beschriebene Verfahren zur Herstellung von Verbindungen der allgemeinen Formel III benötigten Ausgangsverbindungen der allgemeinen Formel IV sind durch Umsetzung von 3,3;17,17-Bis-(ethylendioxy)-5-estren-11-on (oder einer analogen Verbindung mit anderen geeigneten Ketoschutzgruppen) mit dem entsprechenden Perfluoralkylsulfonsäureanhydrid $(C_nF_{2n+1}SO_2)_2O$ $[n=1,2,3,4]$ unter basischen Bedingungen erhältlich (M.E. Wright, S.R. Pulley, J. Org. Chem. 1989, 54, 2886).

Außer in der Form des Ethylendioxyketals können die Ketogruppen in der bereits vorstehend beschriebenen Weise (vgl. allgemeine Formel III) geschützt sein.

Vorzugsweise wird die Trifluormethylsulfonyloxyverbindung zur Gewinnung der Verbindungen der allgemeinen Formel III verwendet.

Als Base wird im Rahmen vorliegender Erfindung insbesondere 2,6-Di-tert.-butylpyridin eingesetzt; es kommen jedoch auch andere Pyridinderivate in Frage.

Die Erfindung wird anhand nachstehender Beispiele näher erläutert:

## Beispiele

### 3,3;17,17-Bis-(ethylendioxy)-11-trifluormethylsulfonyloxy-5,9(11)-estradien

26,1 g (69,7 mmol) 3,3;17,17-Bis-(ethylendioxy)-5-estren-11-on werden in 350ml absolutem Methylenchlorid gelöst und unter Schutzgas mit 18 ml 2,6-Di-tertiär-butylpyridin versetzt. Nach Kühlen dieser

Lösung auf 0 °C werden 12,9 ml (76,8 mmol) Trifluormethansulfonsäureanhydrid langsam zugetropft. Danach wird das Reaktionsgemisch 20 Stunden bei Raumtemperatur nachgerührt. Zur Aufarbeitung wird es auf gesättigte Natriumhydrogencarbonatlösung gegossen, die organische Phase abgetrennt und die wäßrige mit Methylenchlorid nachextrahiert. Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Ethylacetat/Hexan werden neben 16,4 ml 2,6-Ditertiär-butylpyridin und 5,1 g 3,3;17,17-Bis-(ethylendioxy)-5-estren-11-on 27 g 3,3;17,17-Bis-(ethylendioxy)-11-trifluormethylsulfonyloxy-5,9(11)-estradien als weißer Schaum erhalten.

$[\alpha]^{20}_D$ = + 104 ° ($CHCl_3$;c = 0.505)

$^1$H-NMR($CDCl_3$) $\delta$: 5,58 ppm (1H,d breit J = 5Hz,H-6); 3,7-4,0 ppm (8H,m,H-Ketale); 2,88 ppm (1H,d breit J = 11Hz,H-10); 2,74 ppm (1H,dtr J = 16Hz und J = 2,5Hz,H-12); 2,18-2,33 ppm (2H,m,H-4); 0,84 ppm (3H,s,H-18).

3,3;17,17-Bis-(ethylendioxy)-11-trifluormethylsulfonyloxy-5,11-estradien

11,2 ml (80,1 mmol) Diisopropylamin werden bei -30 °C in 260ml absolutem Tetrahydrofuran unter Schutzgas vorgelegt und mit 1,8 ml einer 1,6 molaren n-Butyllithiumlösung in Hexan versetzt. Anschließend wird die Lösung 1 Stunde bei 0 °C gerührt. Nach Zutropfen einer Lösung von 10 g 3,3;17,17-Bis-(ethylendioxy)-5-estren-11-on in 130 ml absolutem Tetrahydrofuran wird zur Deprotonierung 45 Minuten bei 0 °C nachgerührt, dann das Reaktionsgemisch auf -78 °C heruntergekühlt und mit 13 ml Trifluormethansulfonsäureanhydrid durch langsames Zutropfen versetzt. Nach 2,5-stündigem Nachrühren bei -78 °C wird das Reaktionsgemisch auf gesättigte Natriumhydrogencarbonatlösung gegossen und die wäßrige Phase mit Ethylacetat extrahiert. Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Etylacetat/Hexan werden neben 2,4 g 3,3;17,17-Bis-(ethylendioxy)-5-estren-11-on 5,9 g 3,3;17,17-Bis-(ethylendioxy)-11-trifluormethylsulfonyloxy-5,11-estradien als weißer Schaum erhalten.

Fp. = 128-129 °C (Diisopropylether); $[\alpha]^{20}_D$ = - 31 ° ($CHCl_3$;c = 0.505)

3,3;17,17-Bis-(ethylendioxy)-11-(4-methoxyphenyl)-5,9(11)-estradien

a) 21,6 g (42,6 mmol) 3,3;17,17-Bis-(ethylendioxy)-11-trifluormethylsulfonyloxy-5,9(11)-estradien werden in einem Gemisch aus 360 ml Toluol und 170 ml Ethanol gelöst und nacheinander mit 2,5 g Palladiumtetrakistriphenylphosphin, 3,6 g Lithiumchlorid, 55 ml 2 m Natriumcarbonatlösung und 7,2 g (46,8 mmol) 4-Methoxyphenylboronsäure versetzt. Das Reaktionsgemisch wird dann 2 Stunden bei 95 °C gerührt, auf Raumtemperatur abgekühlt und mit gesättigter Natriumchloridlösung versetzt. Die organische Phase wird abgetrennt, nacheinander mit 5 %-iger Natronlauge und Wasser gewaschen, über Natriumsulfat getrocknet und am Vakuum eingeengt. Der Rückstand wird an Kieselgel mit einem Gemisch aus Ethylacetat/Hexan chromatographiert. Man erhält 19,2 g 3,3;17,17-Bis-(ethylendioxy)-11-(4-methoxyphenyl)-5,9(11)-estradien als weißen Schaum.

b) 1,52 g 3,3;17,17-Bis-(ethylendioxy)-11 -trifluormethylsulfonyloxy-5,9(11)-estradien werden in 25 ml absolutem Dimethylformamid gelöst und mit 270 mg Lithiumchlorid und 350 mg Tetrakistriphenylphophinpalladium versetzt. Nach fünfminütigem Nachrühren wird das Reaktionsgemisch mit 1,3 ml Tri-n-butyl-4-methoxyphenylzinn versetzt, 3 Stunden bei 110 °C unter Schutzgas gerührt, auf Raumtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Aluminiumoxid (neutral, Stufe III) mit einem Gemisch aus Ethylacetat/Hexan ergeben 1,15 g 3,3;17,17-Bis-(ethylendioxy)-11-(4-methoxyphenyl)- 5,9(11)-estradien als weißen Schaum.

Exemplarisch ist die Darstellung einiger weiterer Produkte analog den obigen Vorschriften a) oder b) in der nachstehenden Tabelle aufgeführt :

| Aromat (allg. Formel V ) | Produkt | Ausbeute [%] | Physikalische Daten |
|---|---|---|---|
| a) 4-Methoxy-phenylboronsäure<br>oder<br>b) Tri-n-butyl-4-meth-oxyphenylzinn | 3,3;17,17-Bis-(ethylendioxy)-11-(4-methoxyphenyl)-5,9(11)-estradien | 97<br><br>83 | Fp. = 156 °C (Diisopropylether)<br>$[\alpha]_D^{20}$ = - 0,1 ° (CHCl$_3$; c=0.52) |
| a) 4-Methylphenyl-boronsäure | 3,3;17,17-Bis-(ethylendioxy)-11-(4-methylphenyl)-5,9(11)-estradien | 92 | Fp. = 175 °C (Diisopropylether)<br>$[\alpha]_D^{20}$ = - 11 ° (CHCl$_3$; c=0.505) |
| a) Phenyl-boronsäure | 3,3;17,17-Bis-(ethylendioxy)-11-phenyl-5,9(11)-estradien | | Fp. = 189 °C (Diisopropylether)<br>$[\alpha]_D^{20}$ = - 3 ° (CHCl$_3$; c=0.5) |
| a) 4-Bromphenyl-boronsäure | 3,3;17,17-Bis-(ethylendioxy)-11-(4-bromphenyl)-5,9(11)-estradien | 62 | Fp. = 171 °C (Diisopropylether)<br>$[\alpha]_D^{20}$ = - 15 ° (CHCl$_3$; c=0.5) |
| a) 4-(Dimethylami-no)phenylboron-säure | 3,3;17,17-Bis-(ethylendioxy)-11-[4-(dimethylamino)phenyl]-5,9(11)-estradien | 98 | Fp. = 211 °C (Diisopropylether)<br>$[\alpha]_D^{20}$ = - 48 ° (CHCl$_3$; c=0.52) |

3,3;17,17-Bis-(ethylendioxy)-11-(4-methylphenyl)-5,11-estradien

5,5 g (9,9 mmol) 3,3;17,17-Bis-(ethylendioxy)-11-trifluormethylsulfonyloxy-5,11-estradien werden in einem Gemisch aus 90ml Toluol und 40 ml Ethanol gelöst und nacheinander mit 0,6g Palladiumtetrakistriphenylphosphin, 0,85g Lithiumchlorid, 13 ml 2m Natriumcarbonatlösung und 1,5 g (11 mmol) 4-Methylphenylboronsäure versetzt. Das Reaktionsgemisch wird dann 1 Stunde bei 95 °C gerührt, auf Raumtemperatur abgekühlt und mit gesättigter Natriumchloridlösung versetzt. Die organische Phase wird abgetrennt, nacheinander mit 5 %-iger Natronlauge und Wasser gewaschen, über Natriumsulfat gewaschen und am Vakuum eingeengt. Der Rückstand wird an Kieselgel mit einem Gemisch aus Ethylacetat/Hexan chromatographiert. Man erhält 4,27 g 3,3;17,17-Bis-(ethylendioxy)-11-(4-methylphenyl)-5,11-estradien als weißen Schaum.

Exemplarisch ist die Darstellung einiger weiterer Produkte analog der obigen Vorschrift in der nachstehenden Tabelle aufgeführt :

| Aromat | Produkt | Ausbeute [%] | Physikalische Daten |
|---|---|---|---|
| 4-Methylphenyl-boronsäure | 3,3;17,17-Bis-(ethylendioxy)-11-(4-methylphenyl)-5,11-estradien | 88 | Fp. = 152 °C (Diisopropylether) $[\alpha]_D^{20}$ = + 20 ° ($CHCl_3$; c=0.505) |
| 4-Methoxy-phenylboronsäure | 3,3;17,17-Bis-(ethylendioxy)-11-(4-methoxyphenyl)-5,11-estradien | 92 | Fp. = 148 °C (Diisopropylether) $[\alpha]_D^{20}$ = + 22 ° ($CHCl_3$; c=0.505) |
| Phenyl-boronsäure | 3,3;17,17-Bis-(ethylendioxy)-11-phenyl-5,11-estradien | 91 | $^1$H-NMR ($CDCl_3$) δ [ppm] : 7,12-7,4 (5H,m,H-aromatisch); 5,7 (1H, d J=1Hz,H-12); 5,6 (1H,d J=5Hz breit, H-6); 1,0 (3H,s,H-18) |
| 4-Bromphenyl-boronsäure | 3,3;17,17-Bis-(ethylendioxy)-11-(4-bromphenyl)-5,11-estradien | 65 | $^1$H-NMR ($CDCl_3$) δ [ppm] : 7,37 (2H,d J=8,5Hz, H-aromatisch); 7,05 (2H,d=8,5Hz,H-aromatisch); 5,72 (1H,d J=1Hz,H-12); 5,59 (1H,d J=5Hz breit, H-6); 0,96 (3H,s, H-18) |

3,3;17,17-Bis-(ethylendioxy)-11-(4-hydroxyphenyl)-5,9(11)-estradien

9,33 g 3,3;17,17-Bis-(ethylendioxy)-11-(4-methoxyphenyl)-5,9(11)-estradien werden in 100 ml absolutem Dimethylformamid gelöst, mit 5,6g Natriummethanthiolat versetzt und das Reaktionsgemisch 3 Stunden zum Rückfluß erhitzt. Nach Abkühlen wird es auf Wasser gegossen und die wäßrige Phase mit Ethylacetat extrahiert. Die vereinigten organischen Phasen wäscht man mehrmals mit gesättigter Natriumchloridlösung, trocknet sie über Natriumsulfat und engt am Vakuum ein. Der Rückstand wird an Kieselgel mit einem Gemisch aus Ethylacetat/Hexan chromatographiert und man erhält 8,67 g 3,3;17,17-Bis-(ethylendioxy)-11-(4-hydroxyphenyl)-5,9(11)-estradien als weißen Schaum.

Fp. = 168-170 °C (Ethylacetat); $[\alpha]^{20}_D$ = - 11 ° ($CHCl_3$;c = 0,505)

3,3;17,17-Bis-(ethylendioxy)-11-(4-trifluormethylsulfonyloxyphenyl)-5,9(11)-estradien

4,75 g 3,3;17,17-Bis-(ethylendioxy)-11-(4-hydroxyphenyl)-5,9(11)-estradien werden unter Schutzgas zusammen mit 8,45g 4-Dimethylaminopyridin in 160 ml absolutem Methylenchlorid gelöst, auf -78 °C gekühlt und mit 2,5 ml Trifluormethansulfonsäureanhydrid gelöst in 17 ml absolutem Methylenchlorid versetzt. Nach einstündigem Nachrühren wird das Reaktionsgemisch auf gesättigte Natriumhydrogencarbonatlösung gegossen und die wäßrige Phase mehrmals mit Methylenchlorid extrahiert. Die vereinigten organischen Phasen wäscht min mit gesättigter Natriumchloridlösung, trocknet sie über Natriumsulfat und engt am Vakuum ein. Der Rückstand wird an Kieselgel mit einem Gemisch aus Ethylacetat/Hexan chromatographiert und min erhält 4,21 g 3,3;17,17-Bis-(ethylendioxy)-11-(4-trifluormethylsulfonyloxyphenyl)-5,9(11)-estradien.

$^1$H-NMR ($CDCl_3$) $\delta$: 7,23 ppm (4H,s,H-aromatisch); 5,67 ppm (1H,d breit J = 5,5Hz,H-6); 3,74-4,0 ppm (8H,m,H-Ketale); 2,94 ppm (1H,d breit J = 12,5Hz,H-10); 2,85 ppm (1H,dtr J = 16Hz und J = 4Hz,H-12); 0,9 ppm (3H,s,H-18).

3,3;17,17-Bis-(ethylendioxy)-11-[4-(2-propenyl)phenyl)-5,9(11)-estradien

a) 3,6 g 3,3;17,17-Bis-(ethylendioxy)-11-(4-trifluormethylsulfonyloxyphenyl)-5,9(11)-estradien werden in 48 ml absolutem Dioxan gelöst und mit 525 mg Lithiumchlorid und 1,5 g Tetrakistriphenylphosphinpalladium versetzt. Nach fünfminütigem Nachrühren wird das Reaktionsgemisch mit 3,8 ml Tributylallylzinn versetzt, 3 Stunden bei Rückfluß unter Schutzgas gerührt, auf Raumtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Kieselgel mit einem Gemisch aus Ethylacetat/Hexan ergeben 2,48 g 3,3;17,17-Bis-(ethylendioxy)-11-[4-(2-propenyl)phenyl]-5,9(11)-estradien als weißen Schaum.

$^1$H-NMR ($CDCl_3$) $\delta$: 7,03-7,18 ppm (4H,m,H-aromatisch); 5,89-6,05 ppm (1H,m, H- = CH-); 5,64 ppm (1H,d breit J = 5,5Hz,H-6); 4,98-5,1 (2H,m,H-$CH_2$ = ); 3,7-4,0 ppm (8H, m,H-Ketale); 3,37 ppm (2H,d J = 6,5 Hz,H-ar-$CH_2$); 3,01 ppm (1H,d breit J = 10,5Hz,H-10); 2,84 ppm (1H,dtr J = 16Hz und J = 2,5Hz,H-12); 0,92 ppm (3H,s,H-18).

b) 2,05 g 3,3;17,17-Bis-(ethylendioxy)-11-(4-bromphenyl)-5,9(11)-estradien werden in 80 ml absolutem Dioxan gelöst und 200 mg Tetrakistriphenylphosphinpalladium versetzt. Nach fünfminütigem Nachrühren wird das Reaktionsgemisch mit 3,9 ml Tributylallylzinn versetzt, 2 Stunden bei Rückfluß unter Schutzgas gerührt, auf Raumtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Kieselgel mit einem Gemisch aus Ethylacetat/Hexan ergeben 1,71 g 3,3;17,17-Bis-(ethylendioxy)-11-[4-(2-propenyl)phenyl]-5,9(11)-estradien als weißen Schaum.

## Patentansprüche

1. Verbindungen der allgemeinen Formel III

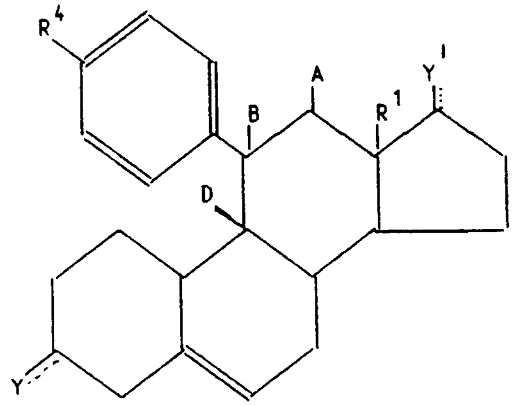

(III),

worin
A und B gemeinsam eine zusätzliche Bindung und
D ein Wasserstoffatom oder
B und D gemeinsam eine zusätzliche Bindung und
A ein Wasserstoffatom
bedeuten und
$R^4$ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten $C_1$-$C_8$-Alkyl-, -Acyl- oder Alkoxyalkylrest, für
eine Aminogruppe

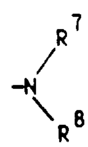

,

in welcher $R^7$ und $R^8$ unabhängig voneinander ein Wasserstoffatom oder eine $C_1$-$C_4$-Alkylgruppe bedeuten, oder für ein entsprechendes Aminoxid

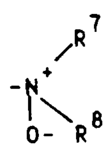

,

oder für die Gruppierungen $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα

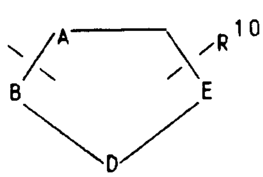

(Iα),

in welchem A ein Stickstoff-, Sauerstoff- oder Schwefelatom, -B-D-E- die Elementenfolge -C-C-C-, -N-C-C- oder -C-N-C- und $R^{10}$ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten $C_1$-$C_8$-Alkyl-, -Acyl-oder Alkoxyalkylrest, für
eine Aminogruppe

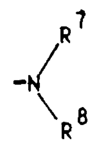

,

in welcher $R^7$ und $R^8$ unabhängig voneinander ein Wasserstoffatom oder eine $C_1$-$C_4$-Alkylgruppe bedeuten, oder ein entsprechendes Aminoxid

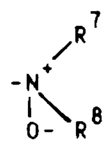

,

oder die Gruppierung $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten,
symbolisieren,
oder für einen Heteroarylrest der Formel Iβ

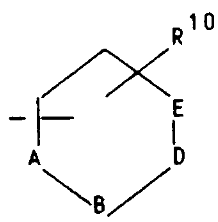

(IB),

in welchem A ein Stickstoffatom und -B-D-E- die Elementenfolge -C-C-C-, -N-C-C-, -C-N-C- oder -C-C-N- bedeuten und $R^{10}$ die bereits angegebene Bedeutung hat,
oder für einen Phenylrest der Formel Iγ

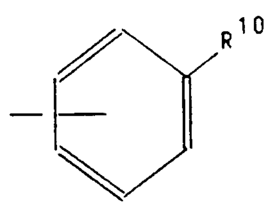

(Iγ),

worin $R^{10}$ die bereits angegebene Bedeutung hat,
stehen,
Y und Y' eine geschützte Ketogruppe oder eine geschützte Hydroxygruppe und ein Wasserstoffatom, wobei Y und Y' identisch oder verschieden sein können sowie
$R^1$ eine Methyl- oder Ethylgruppe bedeuten.

**2.** 3,3;17,17-Bis-(ethylendioxy)-11-(4-methoxyphenyl)-5,9(11)-estradien,
3,3;17,17-Bis-(ethylendioxy)-11-(4-methylphenyl)-5,9(11)-estradien,
3,3;17,17-Bis-(ethylendioxy)-11-phenyl-5,9(11)-estradien,
3,3;17,17-Bis-(ethylendioxy)-11-(4-bromphenyl)-5,9(11)-estradien,
3,3;17,17-Bis-(ethylendioxy)-11-(4-methoxyphenyl)-5,11-estradien,

3,3;17,17-Bis-(ethylendioxy)-11-(4-methylphenyl)-5,11-estradien,
3,3;17,17-Bis-(ethylendioxy)-11-phenyl-5,11-estradien,
3,3;17,17-Bis-(ethylendioxy)-11-(4-bromphenyl)-5,11-estradien.

**3.** Verfahren zur Herstellung von Verbindungen der allgemeinen Formel III

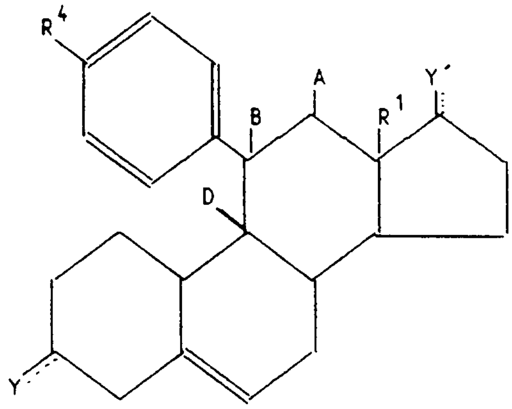

(III),

worin A, B und D sowie Y und Y' , $R^1$ und $R^4$ die bereits in Anspruch 1 angegebene Bedeutung haben, dadurch gekennzeichnet, daß eine Verbindung der allgemeinen Formel IV

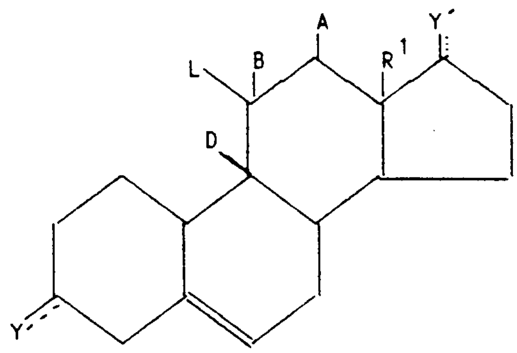

(IV),

worin A, B und D sowie Y und Y' und $R^1$ die in Formel III angegebene Bedeutung haben und L für eine Perfluoralkylsulfonyloxygruppe $C_nF_{2n+1}SO_2O$-(n = 1,2,3,4) steht, in Gegenwart einer katalytischen Menge eines Übergangsmetallkatalysators mit einer Arylverbindung der allgemeinen Formel V

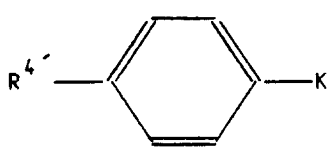

(V),

worin K für einen der Reste

- -B(Alkyl)$_2$
- -Sn(Alkyl)$_3$ Alkyl = $C_1$-$C_4$-Alkylrest
- -B(OH)$_2$
- -ZnHal
- -MgHal Hal = Cl, Br, J

und $R^{4'}$ für einen der unter $R^4$ genannten Reste stehen,
zu einer Verbindung der allgemeinen Formel IIIa

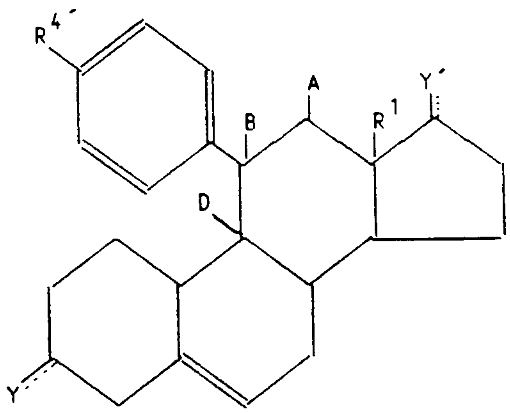

(IIIa),

worin A, B und D sowie Y und Y' und $R^1$ die in Formel III und $R^{4'}$ die in Formel V angegebene Bedeutung haben und gegebenenfalls, wenn $R^4$ in der Formel III eine andere Bedeutung als $R^{4'}$ in der Formel IIIa haben soll, eine Verbindung der allgemeinen Formel IIIa, worin $R^{4'}$ für ein Bromatom steht oder nach Überführung einer für $R^{4'}$ stehenden Methoxygruppe in eine Perfluoralkylsulfonyloxygruppe $C_nF_{2n+1}SO_2O$- (n = 1,2,3,4) mit einer Verbindung der allgemeinen Formel VI

$R^4$-K (VI),

worin $R^4$ die letztlich für diesen Substituenten in der Formel III gewünschte und K die bereits in Formel V angegebene Bedeutung haben,
umgesetzt wird.

4. Verfahren nach Anspruch 3, dadurch gekennzeichnet, daß eine Verbindung der allgemeinen Formel IV, in der für L eine Trifluormethylsulfonyloxygruppe steht, umgesetzt wird.

5. Verfahren nach Anspruch 3, dadurch gekennzeichnet, daß mit Palladiumtetrakistriphenylphosphin als Übergangsmetallkatalysator umgesetzt wird.

6. Ausgangsverbindungen der allgemeinen Formel IV

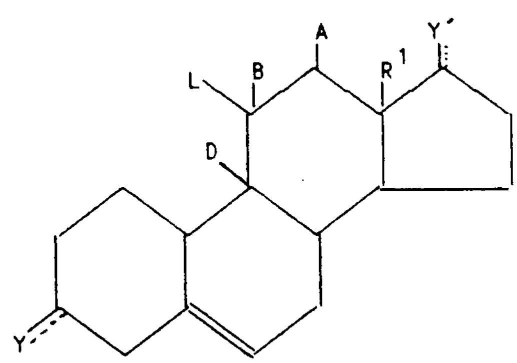

(IV),

worin
A und B gemeinsam eine zusätzliche Bindung und
D ein Wasserstoffatom oder
B und D gemeinsam eine zusätzliche Bindung und
A ein Wasserstoffatom,
L eine Perfluoralkylsulfonyloxygruppe $C_nF_{2n+1}SO_2O$- (n = 1, 2, 3, 4),
$R^1$ eine Methyl- oder Ethylgruppe und
Y und Y' eine geschützte Ketogruppe oder eine geschützte Hydroxygruppe und ein Wasserstoffatom, wobei Y und Y' identisch oder verschieden sein können,
bedeuten.

## Claims

**1.** Compounds of the general formula III

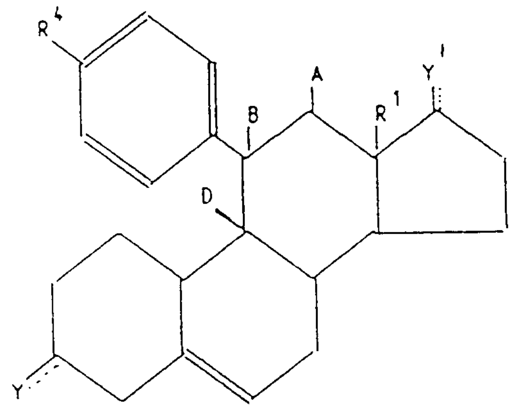

(III)

wherein
A and B together represent an additional bond and
D represents a hydrogen atom or
B and D together represent an additional bond and
A represents a hydrogen atom, and
$R^4$ represents a hydrogen atom, a cyano group, a chlorine, fluorine, bromine or iodine atom, a trialkylsilyl or trialkylstannyl group, a straight-chain or branched, saturated or unsaturated $C_1$-$C_8$alkyl, -acyl or alkoxyalkyl radical, an amino group

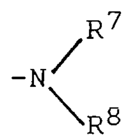

,

in which $R^7$ and $R^8$ each represents, independently of the other, a hydrogen atom or a $C_1$-$C_4$alkyl group, a corresponding amine oxide

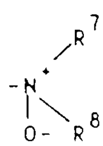

or a group $-OR^9$ or $-S(O)_iR^9$ in which i = 0, 1 or 2 and $R^9$ represents a hydrogen atom, a methyl, ethyl, propyl, isopropyl, methoxyphenyl, allyl or 2-dimethylaminoethyl group, or $R^4$ represents a heteroaryl radical of formula Iα

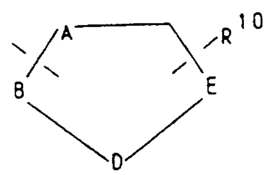

(Iα)

in which A represents a nitrogen, oxygen or sulphur atom, -B-D-E- represents the element sequence -C-C-C-, -N-C-C-or -C-N-C- and $R^{10}$ represents a hydrogen atom, a cyano group, a chlorine, fluorine, bromine or iodine atom, a trialkylsilyl or trialkylstannyl group, a straight-chain or branched, saturated or

unsaturated $C_1$-$C_8$alkyl, -acyl or alkoxyalkyl radical, an amino group

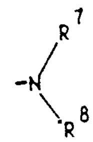

in which $R^7$ and $R^8$ each represents, independently of the other, a hydrogen atom or a $C_1$-$C_4$alkyl group, or a corresponding amine oxide

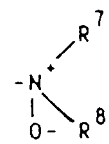

or a group $-OR^9$ or $-S(O)_iR^9$ in which i = 0, 1 or 2 and $R^9$ represents a hydrogen atom, a methyl, ethyl, propyl, isopropyl, methoxyphenyl, allyl or 2-dimethylaminoethyl group, or
$R^4$ represents a heteroaryl radical of formula I$\beta$

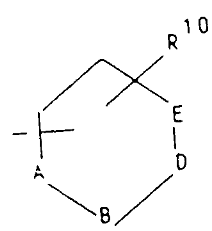

(Iβ)

in which A represents a nitrogen atom and -B-D-E- represents the element sequence -C-C-C-, -N-C-C-, -C-N-C- or -C-C-N- and $R^{10}$ has the meanings already given or $R^4$ represents a phenyl radical of the formula I$\gamma$

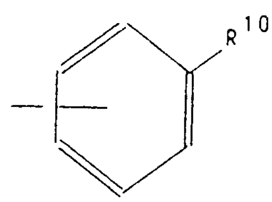

(Iγ)

in which $R^{10}$ has the meanings already given,
Y and Y' represent a protected keto group or a protected hydroxy group and a hydrogen atom, it being possible for Y and Y' to be the same or different, and $R^1$ represents a methyl or ethyl group.

**2.** 3,3;17,17-bis-(ethylenedioxy)-11-(4-methoxyphenyl)-5,9,(11)-oestradiene,
3,3;17,17-bis-(ethylenedioxy)-11-(4-methylphenyl)-5,9,(11)-oestradiene,
3,3;17,17-bis-(ethylenedioxy)-11-phenyl-5,9,(11)-oestradiene,
3,3;17,17-bis-(ethylenedioxy)-11-(4-bromophenyl)-5,9,(11)-oestradiene,
3,3;17,17-bis-(ethylenedioxy)-11-(4-methoxyphenyl)-5,11-oestradiene,
3,3;17,17-bis-(ethylenedioxy)-11-(4-methylphenyl)-5,11-oestradiene,
3,3;17,17-bis-(ethylenedioxy)-11-phenyl-5,11-oestradiene,
3,3;17,17-bis-(ethylenedioxy)-11-(4-bromophenyl)-5,11-oestradiene.

**3.** Process for the preparation of compounds of the general formula III

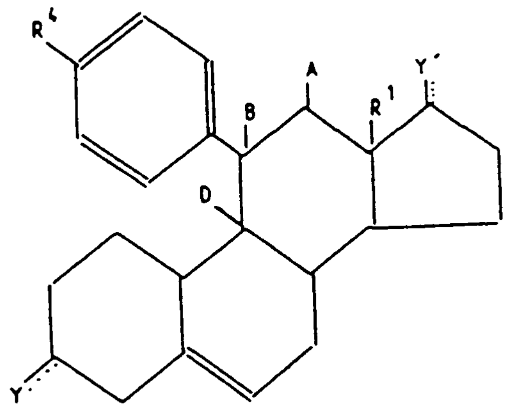

(III)

wherein A, B and D as well as Y and Y', $R_1$ and $R_4$ are as defined for claim 1, characterised in that a compound of the general formula IV

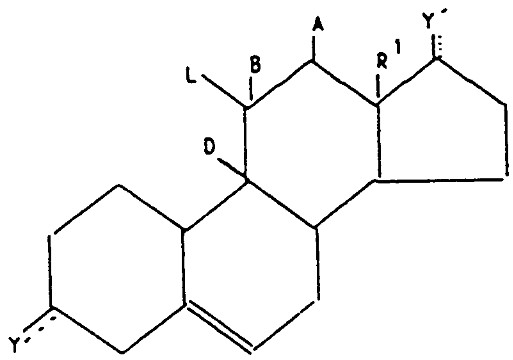

(IV)

wherein A, B and D and also Y and Y' and $R^1$ are as defined for formula III and L represents a perfluoroalkylsulphonyloxy group $C_nF_{2n+1}SO_2O$- (n = 1, 2, 3, 4), is reacted, in the presence of a catalytic amount of a transition metal catalyst, with an aryl compound of the general formula V

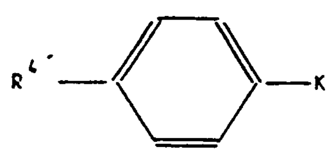

(V)

wherein K represents one of the radicals

-$B(alkyl)_2$

-$Sn(alkyl)_3$ alkyl = a $C_1$-$C_4$ alkyl radical

-$B(OH)_2$

-ZnHal

-MgHal Hal = Cl, Br, I,

and $R^{4'}$ represents one of the radicals mentioned under $R^4$, to form a compound of the general formula IIIa

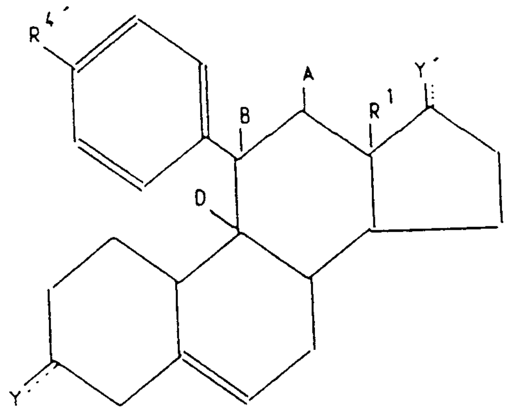

(IIIa)

wherein A, B and D and also Y and Y' and $R^1$ are as defined for formula III and $R^{4'}$ is as defined for formula V, and optionally, if $R^4$ in formula III is to have a meaning different from that of $R^{4'}$ in formula IIIa, a compound of the general formula IIIa wherein $R^{4'}$ represents a bromine atom, or, after conversion of a methoxy group $R^{4'}$, a perfluoroalkylsulphonyloxy group $C_nF_{2n+1}SO_2O$- (n = 1, 2, 3, 4), is reacted with a compound of the general formula VI

$R^4$-K (VI)

wherein $R^4$ has the meaning ultimately desired for that substituent in formula III and K is as defined for formula V.

**4.** Process according to claim 3, characterised in that the reaction is carried out with a compound of the general formula IV in which L represents a trifluoromethylsulphonyloxy group.

**5.** Process according to claim 3, characterised in that the reaction is carried out with palladium tetrakistriphenylphosphine as transition metal catalyst.

**6.** Starting compounds of the general formula IV

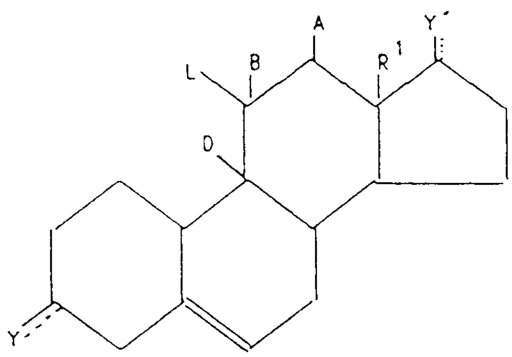

(IV)

wherein
A and B together represent an additional bond and
D represents a hydrogen atom or
B and D together represent an additional bond and
A represents a hydrogen atom,
L represents a perfluoroalkylsulphonyloxy group
$C_nF_{2n+1}SO_2O$- (n = 1, 2, 3, 4),
$R^1$ represents a methyl or ethyl group and
Y and Y' represent a protected keto group or a protected hydroxy group and a hydrogen atom, it being possible for Y and Y' to be the same or different.

## Revendications

**1.** Composés de formule générale III ci-dessous

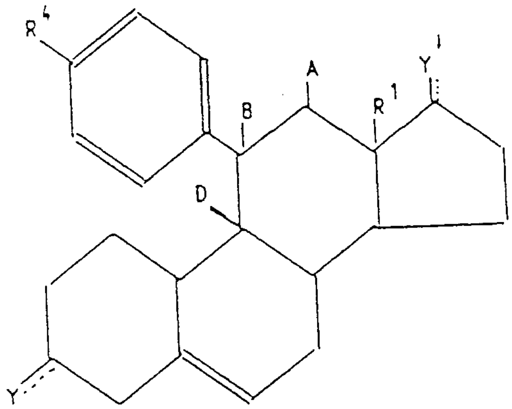

(III),

dans laquelle
A et B représentent ensemble une liaison supplémentaire et
D un atome d'hydrogène ou bien
B et D une liaison supplémentaire et
A un atome d'hydrogène,
$R^4$ représente un atome d'hydrogène, un groupe cyano, un atome de chlore, de fluor, de brome ou d'iode, un groupe trialkylsilyle ou trialkylstannyle, un $C_1$-$C_8$-alkyl, -acyl-ou -alcoxyalkyle linéaire ou ramifié saturé ou insaturé, un groupe amino

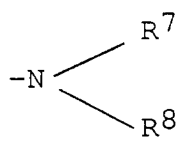

dont $R^7$ et $R^8$ sont chacun, indépendamment l'un de l'autre, un atome d'hydrogène ou un alkyle en $C_1$-$C_4$, ou un aminoxyde

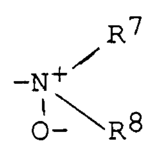

correspondant, ou bien un groupe $-OR^9$ ou $-S(O)_iR^9$ avec i = 0, 1 ou 2 et $R^9$ un atome d'hydrogène ou un groupe méthyle, éthyle, propyle, isopropyle, méthoxyphényle, allyle ou 2-diméthylaminoéthyle, ou un hétéroaryle de formule Iα

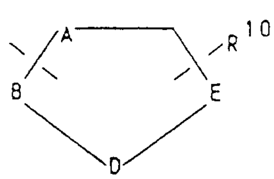

(Iα),

dans laquelle A désigne un atome d'azote, d'oxygène ou de soufre, -B-D-E- la suite -C-C-C-, -N-C-C- ou -C-N-C- et $R^{10}$ un atome d'hydrogène, un groupe cyano, un atome de chlore, de fluor, de brome ou

d'iode, un groupe trialkyl-silyle ou trialkyl-stannyle, un $C_1$-$C_8$-alkyl-, -acyl- ou alcoxyalkyle linéaire ou ramifié, saturé ou insaturé, ou bien un groupe amino

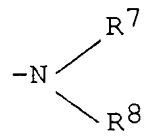

dont $R^7$ et $R^8$ sont chacun,
indépendamment l'un de l'autre, un atome d'hydrogène ou un alkyle en $C_1$-$C_4$, ou un aminoxyde correspondant

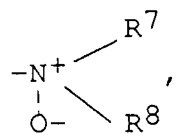

ou encore un groupe -$OR^9$ ou -$S(O)_iR^9$ avec i = 0, 1 ou 2 et $R^9$ un atome d'hydrogène ou un groupe méthyle, éthyle, propyle, isopropyle, méthoxyphényle, allyle ou 2-diméthylaminoéthyle, ou un hétéroaryle de formule $I\beta$

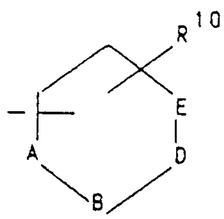

(IB),

dans laquelle A désigne un atome d'azote, -B-D-E- la suite -C-C-C-, -N-C-C-, -C-N-C- ou -C-C-N- et $R^{10}$ a la signification précédemment indiquée, ou bien un phényle de formule de formule $I\gamma$

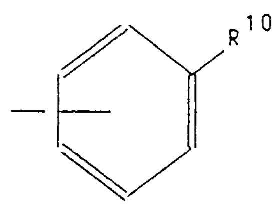

($I\gamma$),

$R^{10}$ ayant la signification précédemment indiquée,
Y et Y' représentent un groupe céto protégé ou bien un hydroxyle protégé et un atome d'hydrogène et peuvent être identiques ou différents l'un de l'autre, et
$R^1$ le groupe méthyle ou éthyle.

**2.** 3,3;17,17-Bis-(éthylène-dioxy)-11-phényl-5,11-estradiène,
3,3;17,17-Bis-(éthylène-dioxy)-11-(4-bromophényl)-5,11-estradiène
3,3;17,17-Bis-(éthylène-dioxy)-11-(4-méthoxyphényl)-5,9(11)-estradiène
3,3;17,17-Bis-(éthylène-dioxy)-11-(4-méthylphényl)-5,9(11)-estradiène
3,3;17,17-Bis-(éthylène-dioxy)-11-phényl-5,9(11)-estradiène 3,3;17,17-Bis-(éthylène-dioxy)-11-(4-bromophényl)-5,9(11)-estradiène
3,3;17,17-Bis-(éthylène-dioxy)-11-(4-méthoxyphényl)-5,11-estradiène
3,3;17,17-Bis-(éthylène-dioxy)-11-(4-méthylphényl)-5,11-estradiène.

**3.** Procédé de préparation des composés de formule III telle que définie à la revendication 1, procédé caractérisé en ce que l'on fait réagir un composé de formule générale IV

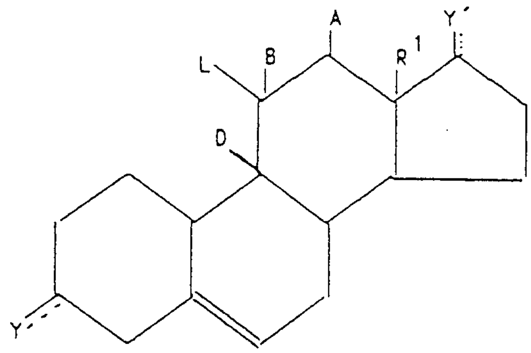

(IV),

les divers symboles ayant les mêmes significations que dans la formule III et L désignant un groupe perfluoralkylsulfonyloxy $C_nF_{2n+1}SO_2O$- (n = 1,2,3 ou 4), en présence d'une proportion catalytique d'un catalyseur de métal de transition, avec un composé arylique de formule V

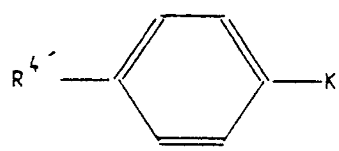

(V),

K étant l'un des radicaux :
-B(alkyle)$_2$
-Sn(alkyle)$_3$ Alkyle = $C_1$-$C_4$-alkyle
-B(OH)$_2$
-ZnHal
-MgHal Hal = Cl, Br ou I
et $R^{4'}$ l'un des radicaux indiqués pour $R^4$,
pour former un composé de formule IIIa

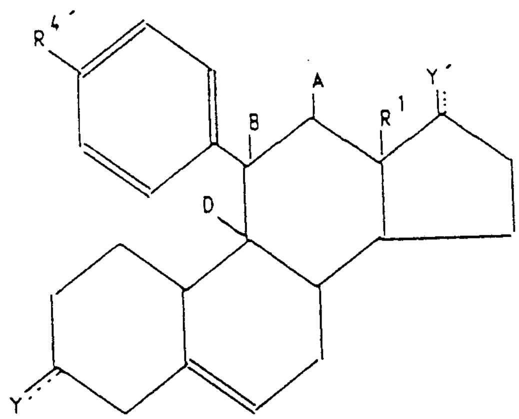

(IIIa),

dans laquelle les divers symboles ont les mêmes significations que dans la formule III et $R^{4'}$ les mêmes significations que dans la formule V, et le cas échéant, si $R^4$ doit avoir dans la formule III une autre signification que $R^{4'}$ dans la formule IIIa, on fait réagir un composé de formule IIIa dont $R^{4'}$ est un atome de brome, ou après transformation d'un groupe méthoxy $R^{4'}$ en un groupe perfluoralkylsulfonyloxy $C_nF_{2n+1}SO_2O$- (n = 1,2,3 ou 4), avec un composé de formule VI

$R^4$-K (VI)

$R^4$ ayant la signification finalement voulue pour ces substituants dans la formule III et K la signification indiquée pour la formule V.

**4.** Procédé selon la revendication 3, caractérisé en ce que l'on fait réagir un composé de formule IV dont L est un groupe trifluorométhylsulfonyloxy.

**5.** Procédé selon la revendication 3, caractérisé en ce que l'on effectue la réaction avec la palladiumtétrakistriphénylphosphine comme catalyseur de métal de transition.

**6.** Les composés de formule générale IV

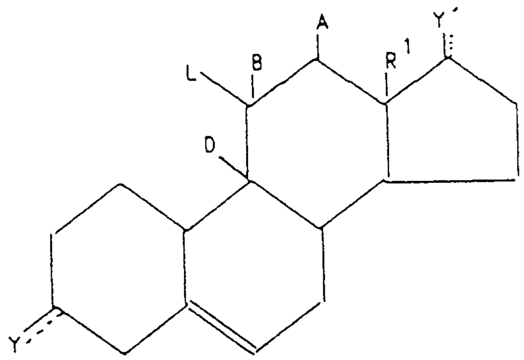

(IV),

dans laquelle
A et B représentent ensemble une liaison supplémentaire et
D un atome d'hydrogène, ou bien
B et D une liaison supplémentaire et
A un atome d'hydrogène,
L est un groupe perfluoralkylsulfonyloxy $C_nF_{2n+1}SO_2O$-(n = 1,2,3 ou 4),
$R^1$ le groupe méthyle ou éthyle et
Y et Y' sont un groupe céto protégé ou un groupe hydroxy protégé et un atome d'hydrogène et sont identiques ou différents l'un de l'autre.